# EUROPEAN PATENT SPECIFICATION

(11) **EP 1 727 562 B1**
(45) Date of publication and mention of the grant of the patent: **18.06.2008**
(21) Application number: 05708706.6
(22) Date of filing: 04.03.2005
(51) Int. Cl.: A61K 39/12, A61K 39/295

(54) **METHOD OF VACCINATION AGAINST TESTICULAR BVDV INFECTION**
IMPFVERFAHREN GEGEN TESTIKULÄRE BVDV-INFEKTION
METHODE DE VACCINATION CONTRE UNE INFECTION BVDV TESTICULAIRE

(30) Priority: 17.03.2004 US 553867 P
(43) Date of publication of application: 06.12.2006
(73) Proprietor: Pharmacia & Upjohn Company LLC, Kalamazoo, MI 49001 (US)
(72) Inventor: ELLSWORTH, Michael, Aaron c/o Pfizer Inc., Lincoln, NE 68521 (US); TUCKER, Cassius, McAllister Pfizer Inc., Kalamazoo, MI 49001 (US)
(74) Representative: Courgeon, Antoine
(86) International application number: PCT/IB2005/000610
(87) International publication number: WO 2005/089793

(56) References cited:
- FRAY M D ET AL: "The effects of bovine viral diarrhoea virus on cattle reproduction in relation to disease control" ANIMAL REPRODUCTION SCIENCE, vol. 60-61, no. Special Issue, 2 July 2000 (2000-07-02), pages 615-627, XP002331833 ISSN: 0378-4320
- VAN OIRSCHOT J T ET AL: "Vaccination of cattle against bovine viral diarrhoea" VETERINARY MICROBIOLOGY, vol. 64, no. 2-3, January 1999 (1999-01), pages 169-183, XP002331834 ISSN: 0378-1135
- BOLIN S R: "Control of bovine viral diarrhea infection by use of vaccination." THE VETERINARY CLINICS OF NORTH AMERICA. FOOD ANIMAL PRACTICE. NOV 1995, vol. 11, no. 3, November 1995 (1995-11), pages 615-625, XP009049066 ISSN: 0749-0720 cited in the application
- GIVENS M D ET AL: "BOVINE VIRAL DIARRHEA VIRUS IN EMBRYO AND SEMEN PRODUCTION SYSTEMS" VETERINARY CLINICS OF NORTH AMERICA. FOOD ANIMAL PRACTICE, SAUNDERS, PHILADELPHIA, PA, US, vol. 20, no. 1, March 2004 (2004-03), pages 21-38, XP009047210 ISSN: 0749-0720

## Description

### FIELD OF THE INVENTION

The methods of the invention relate to methods for preventing testicular infection by bovine viral diarrhea virus by immunizing susceptible male animals against infection.

### BACKGROUND OF THE INVENTION

Bovine viral diarrhea virus (BVDV) is an economically significant pathogen of cattle and other susceptible animals that can be shed in the semen of persistently and acutely infected bulls and other susceptible male animals. This pathogen causes gastrointestinal, respiratory and reproductive disease in susceptible animals.

While gastrointestinal and respiratory disease due to highly pathogenic strains of BVDV are more clinically dramatic, reproductive losses due to BVDV can be much more economically significant. The literature on transmission of BVDV has established that the virus in the semen of bulls can infect susceptible, inseminated cows, causing reduced pregnancy rates, early embryonic death, abortion, and birth of calves persistently infected with BVDV.¹⁻³

Persistently-infected bulls most consistently infect susceptible inseminated cows because their semen contains a high concentration of virus (10^{7.6} cell culture infective doses (50%)/mL) (CCID₅₀/mL).³ In comparison, infected testes of acutely infected immunocompetent bulls shed lower concentrations of virus (5 to 75 CCID₅₀/mL) in semen, but they also are capable of transmitting BVDV.⁴ One study reported that 25 to 50 CCID₅₀/mL of virus in semen infected 5% of inseminated heifers, and subsequent horizontal transmission of BVDV from the infected heifers to pregnant herdmates resulted in persistent infection of their fetuses.⁵

Two teams of investigators have also reported that acute infection of postpuberal bulls can cause a persistent BVDV infection that localizes in the testes.^{6,7} One unique case occurred in a seropositive, nonviremic bull maintained in an artificial insemination station in New Zealand. The bull had been admitted to the artificial insemination center after attempts at isolating BVDV from blood were negative. Despite the presence of neutralizing antibodies to BVDV, the bull continuously shed virus in semen at low levels (2 x 10³ CCID₅₀/mL) for 11 months when the animal was sacrificed. Source of the acute infection was unknown. On postmortem examination, virus was isolated only from the bull's testes.⁶ Virus in the semen of this bull resulted in infection and subsequent seroconversion of 1 of 3 inseminated seronegative heifers.⁸ In a second artificially induced infection, BVDV was detected by reverse transcription-nested polymerase chain reaction (RT-nPCR) in the semen of 2 of 3 nonviremic postpuberal bulls for up to 7 months, but the virus could not be isolated by standard tissue culture methods. Semen collected 5 months after the initial exposure caused BVDV infection in one seronegative calf following intravenous administration. Semen collected on the day of exposure and 7 months after exposure did not cause infection in two additional seronegative calves.⁷

Two studies have noted that acutely infected bulls can shed BVDV in semen with acceptable spermatozoa concentration, motility, and morphology.^{4,5} Another study found a decrease in motility, an increase in diadem defects, small spermatozoal heads and proximal droplets in acutely infected bulls.⁹

Regardless of any uncertainty about details as to transmissibility by natural breeding and effect on bull fertility, it is clear that testicular infection of susceptible male animals by BVDV has a significant economic impact by virtue of reproductive losses due to BVDV transmission to cows.

Testicular viral infections pose a significant challenge for treatment or prevention via immunization because the testicles are known to be immunologically sequestered. Surprisingly we have shown that immunization is an effective means of controlling BVDV testicular infection among susceptible animals.

### REFERENCES

1. Meyling A, Jensen AM. Transmission of bovine virus diarrhea virus (BVDV) by artificial insemination (AI) with semen from a persistently-infected bull. Veterinary Microbiology 1988; 17:97-105.
2. Kirkland PD, Mackintosh SG, Moyle A. The outcome of widespread use of semen from a bull persistently infected with pestivirus. Veterinary Record 1994; 135:527-529.
3. McGowan MR, Kirkland PD. Early reproductive loss due to bovine pestivirus infection. British Veterinary Journal 1995;151:263-270.
4. Kirkland PD, Richards SG, Rothwell JT, et al. Replication of bovine viral diarrhea virus in the bovine reproductive tract and excretion of virus in semen during acute and chronic infections. Veterinary Record 1991;128:587-590.
5. Kirkland PD, McGowan MR, Mackintosh SG, et al. Insemination of cattle with semen from a bull transiently infected with pestivirus. Veterinary Record 1997;140:124-127.
6. Voges H, Homer GW, Rowe S, et al. Persistent bovine pestivirus infection localized in the testes of an immuno-competent, non-viraemic bull. Veterinary Microbiology 1998;61:165-175.
7. Givens MD, Heath AM, Brock KV, et al. Detection of bovine viral diarrhea virus in semen obtained from inoculation of seronegative postpubertal bulls. AJVR 2003;64:428-434.
8. Niskanen R, Alenius S, Belak K, et al. Insemination of susceptible heifers with semen from a nonviraemic bull with persistent bovine virus diarrhea virus infection localized in the testes. Reproduction in Domestic Animals 2002;37:171-175.
9. Paton DJ, Goodey R, Brockman S, et al. Evaluation of the quality and virological status of semen from bulls acutely infected with BVDV. Veterinary Record 1989;124:63-64.

### BRIEF DESCRIPTION OF THE FIGURES

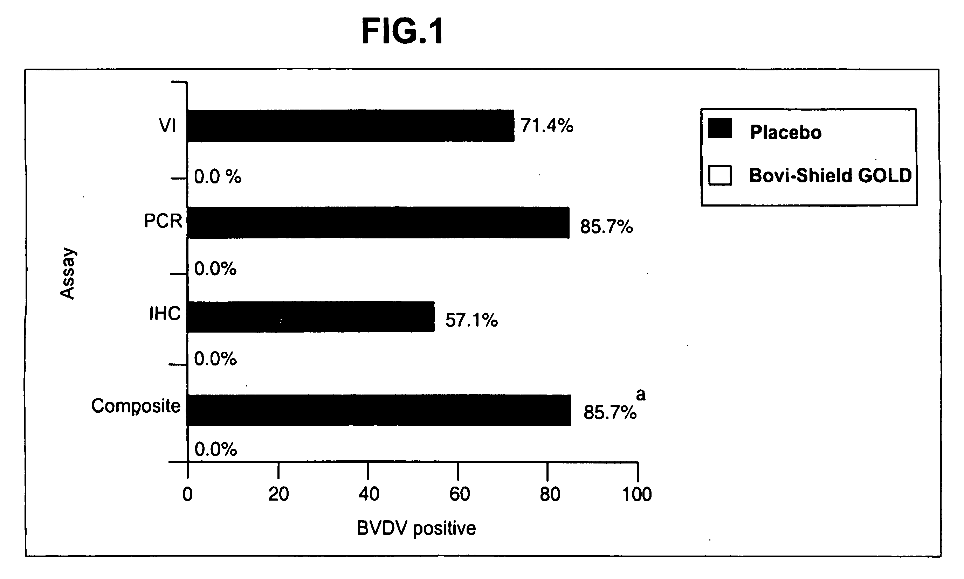

Fig. 1- Percent of testicular biopsy specimens positive for BVDV following type 2 BVDV challenge. Legend: VI = virus isolation, PCR = polymerase chain reaction, IHC = immunohistochemistry a,b Percents with different lower case superscript letters are significantly (P ≤ 0.05) different.

### SUMMARY OF THE INVENTION

The invention comprises a method of preventing or treating testicular BVDV infection in a susceptible male animal comprising administering to the animal an effective amount of a vaccine selected from the group consisting of an inactivated type 1 BVDV vaccine, an inactivated type 2 BVDV vaccine, a modified live type 1 BVDV vaccine, and a modified live type 2 BVDV vaccine.

The invention also comprises a vaccine selected from the group consisting of an inactivated type 1 BVDV vaccine, an inactivated type 2 BVDV vaccine, a modified live type 1 BVDV vaccine, and a modified live type 2 BVDV vaccine for use as a medicament for preventing testicular BVDV infection in a susceptible male animal

The invention further comprises the use of a vaccine selected from the group consisting of an inactivated type 1 BVDV vaccine, an inactivated type 2 BVDV vaccine, a modified live type 1 BVDV vaccine, and a modified live type 2 BVDV vaccine for manufacture of a medicament for preventing testicular BVDV infection in a susceptible male animal.

The invention further comprises a method of preventing testicular BVDV infection in a susceptible male animal comprising identifying an animal with an increased risk of BVDV testicular infection; and administering to the animal an effective amount of a vaccine selected from the group consisting of an inactivated type 1 BVDV vaccine, an inactivated type 2 BVDV vaccine, a modified live type 1 BVDV vaccine, and a modified live type 2 BVDV vaccine.

The invention also comprises a vaccine selected from the group consisting of an inactivated type 1 BVDV vaccine, an inactivated type 2 BVDV vaccine, a modified live type 1 BVDV vaccine, and a modified live type 2 BVDV vaccine for use as a medicament for preventing testicular BVDV infection in a susceptible male animal at increased risk of BVDV testicular infection.

The invention further comprises the use of a vaccine selected from the group consisting of an inactivated type 1 BVDV vaccine, an inactivated type 2 BVDV vaccine, a modified live type 1 BVDV vaccine, and a modified live type 2 BVDV vaccine for manufacture of a medicament for preventing testicular BVDV infection in a susceptible male animal at increased risk of BVDV testicular infection

The invention also comprises an article of manufacture comprising a vessel or vessels containing a BVDV vaccine selected from the group consisting of an inactivated type 1 BVDV vaccine, an inactivated type 2 BVDV vaccine, a modified live type 1 BVDV vaccine, and a modified live type 2 BVDV; and instructions for use of the BVDV vaccine for the prevention of testicular BVDV infection in a susceptible male animal.

The susceptible male animals may include bulls, rams and boars. In a preferred embodiment the animal is a bull.

The vaccines and articles of manufacture may comprise both a modified live type 1 BVDV vaccine and a modified live type 2 BVDV vaccine.

The vaccines may be derived from a cytopathogenic or non-cytopathogenic virus.

Optionally the vaccines and articles of manufacture of the invention may comprises at least one additional antigen selected from the group consisting of Bovine Herpes Virus (BHV-1); Parainfluenza Virus Type 3 (PIV3); . Bovine Respiratory Syncytial Virus (BRSV); *Leptospira canicola, Leptospira grippotyphosa, Leptospira borgpetersenii hardio-prajitno, Leptospira icterohaemmorrhagia, Leptospira interrogans pomona, Leptospira borgpetersenii hardjo-bovis, Leptospira Bratislava, Campylobacter fetus, Mannheimia (Pasteurella) haemolytica, Pasteurella multocida, Mycobacterium bovis,* and *Mycobacterium dispar.*

In a preferred embodiment the vaccines and articles of manufacture of the invention may comprises at least one additional antigen selected from the group consisting of Bovine Herpes Virus (BHV-1), Parainfluenza Virus Type 3 (PIV3), and Bovine Respiratory Syncytial Virus (BRSV).

### DETAILED DESCRIPTION OF THE INVENTION

The present invention provides a method of treating or preventing testicular infection and resultant shedding in semen by BVDV viruses in a susceptible male animal. The method of the present invention is effective in preventing or reducing testicular infections caused by infections caused by types 1 and 2 BVDV.

### DEFINITIONS AND ABBREVIATIONS

"Bovine viral diarrhea virus" ("BVDV") is a small, positive-sense, single stranded, RNA virus in the family Flaviviridae and genus Pestivirus. Two biotypes of BVDV, cytopathic (CP) and noncytopathic (NC), have been described based on the presence or absence of visible cytopathic effect in vitro when susceptible cell monolayers are infected. The noncytopathic biotype is isolated from field outbreaks in a vast majority of cases. Bovine viral diarrhea virus strains can also be categorized into 2 separate species (i.e. genotypes), type 1 and type 2, based on substantial differences within the viral RNA.

The term "susceptible animal" means any animal that is susceptible to BVDV infections for example cattle sheep and pigs.

The term "susceptible male animal" means any male animal that is susceptible to BVDV testicular infections for example male cattle sheep and pigs. Uncastrated male cattle are referred to as "bulls". Uncastrated male sheep are referred to as "rams". Uncastrated male pigs are referred to as "boars".

The term "preventing" or "controlling" with respect to testicular infection means reducing or eliminating the risk of infection by a virulent types 1 and 2 BVDV of the testicles of a susceptible male animal; ameliorating or alleviating the symptoms of an infection, or accelerating the recovery from an infection. The vaccination is considered therapeutic if there is a reduction in viral or bacterial load as assessed by testicular biopsy or presence of virus in semen.

The term "infection" can mean acute or persistent infection with BVDV.

"Acute" or "transient" infection with BVDV occurs when an immunocompetent susceptible animal is exposed to a cytopathic or noncytopathic strain of BVDV. While subclinical infection is most common, signs such as depression, inappetence, oral erosions and ulcerations, diarrhea and death might be observed. An acutely infected immunocompetent animal can transmit the virus to susceptible animals, but much less efficiently than persistently infected animals.

An acute testicular infection refers to an acute or transient infection of the testicles of a susceptible male animal as the result of transient systemic infection. Some reports indicate that an acutely infected bull can shed virus in semen with acceptable concentration, motility and morphology of spermatozoa. However in other reports authors have observed a decrease in motility of spermatozoa and an increase in diadem defects, small spermatozoal heads and proximal droplets coinciding with acute infection.

A persistent infection with BVDV occurs when a susceptible animal is infected with a noncytopathic strain of BVDV before the development of immunocompetency at approximately 125 days of gestation. Persistently infected animals develop immunotolerance to the strain with which they have been infected, act as a pathogen reservoir and commonly shed large quantities of virus in urine, feces, semen, saliva, tears and nasal mucous throughout life.

A persistent testicular infection refers to an persistent infection of the testicles of a susceptible male animal as the result of acute or persistent systemic infection.

### Vaccines used in the Invention

A vaccine used in the invention comprises types 1 and/or 2 BVDV and a veterinary acceptable carrier.
Traditionally, viral vaccines fall into two classes:

Live vaccines containing live -viruses which have been treated or grown in such a way as to make them less- pathogenic (attenuated), and vaccines containing killed (inactivated) virus particles. In the context of BVDV, the viruses themselves may be cytopathogenic or non- cytopathogenic. Bovine viral diarrhea virus strains can also be categorized into 2 separate species (i.e. genotypes), type 1 and type 2, based on substantial differences within the viral RNA. Thus, in principle, eight main classes of BVDV vaccine could exist, although the vast majority of commercial vaccines are based on cytopathogenic viruses.

Among the BVDV vaccines that are currently commercially available are those in which virus has been chemically inactivated (McClurkin, et al., Arch. Virol. 58:119 (1978); Fernelius, et al., Am. J. Vet. Res. 33:1421-1431 (1972); and Kolar, et al., Am. J. Vet. Res. 33:1415-1420 (1972)). These vaccines have typically required the administration of multiple doses to achieve primary immunization, provide immunity of short duration and do not protect against fetal transmission (Bolin, Vet. Clin. North Am. Food Anim. Pract. 11:615-625 (1995)). In sheep, a subunit vaccine based upon a purified E2 protein has been reported (Bruschke, et al., Vaccine 15:1940-1945 (1997)). Unfortunately, only one such vaccine appears to protect fetuses from infection and this protection is limited to one strain of homologous virus.

In addition, modified live virus (MLV) vaccines have been produced using BVD virus that has been attenuated by repeated passage in bovine or porcine cells (Coggins, et al., Cornell Vet. 51:539 (1961); and Phillips, et al., Am. J. Vet Res. 36:135 (1975)) or by chemically induced mutations that confer a temperature-sensitive phenotype on the virus (Lobmann, et al., Am. J. Vet. Res. 45:2498 (1984); and Lobmann, et al., Am. J. Vet. Res. 47:557-561 (1986)). A single dose of MLV vaccine has proven sufficient for immunization and the duration of immunity can extend for years in vaccinated cattle (Coria, et al., Can. J. Con. Med. 42:239 (1978)). In addition, cross-protection has been reported from calves vaccinated with MLV-type vaccines (Martin, et al., In Proceedings of the Conference Res. Workers' Anim. Dis., 75:183 (1994)). However, safety considerations, such as possible fetal transmission of the virus, have been a major concern with respect to the use of these vaccines (Bolin, Vet. Clin. North Am. Food Anim. Pract. 11:615-625 (1995)).

In a preferred embodiment the type 1 BVDV component is modified live cytopathogenic (cpBVD-1 strain NADL-National Animal Disease Center, United States, Dep. of Agriculture, Ames, Iowa, ATCC VR-534). In another preferred embodiment the type 2 BVDV component is modified live cytopathic (cp BVD-2 strain 53637, ATCC No. PTA-4859). As described in copending US Patent Application 60/490,834, filed 7/29/03, both isolates contain an insertion in the NS2-3 region. The attenuated cp BVDV-1 contains an insertion of a *Bos taurus* DnaJ1 coding sequence 3' of the thymidine at nucleotide position # 4993 (NADL sequence numbering), which is the third nucleotide of the codon encoding the glycine residue at amino acid position 1536. The attenuated cp BVDV-2 contains an insertion of a *Bos taurus* DnaJ 1 coding sequence at the same genomic site. In another preferred embodiment, the modified live antigens are desiccated, lyophilized or vitrified.

In one embodiment, the vaccine compositions of the present invention include an effective amount of one or more of the above-described BVD viruses, preferably cpBVD-1 strain NADL (cpBDV-1 strain NADL-National Animal Disease Center, United States Department of Agriculture, Ames, Iowa, ATCC VR-534); cpBVD-2 strain 53637 (ATCC No. PTA-4859), IBRV strain C-13 (Cutter Laboratories); PIV3 strain Reisinger (Univ. Nebraska); BRSV strain 375 (Veterinary Medical Research Institute, Ames, Iowa). Purified BVD viruses can be used directly in a vaccine composition, or preferably, BVD viruses can be further modified by way of serial passages *in vitro.* Typically, a vaccine contains between about 1 x 10² and about 1 x 10¹⁰ plaque forming or TCID₅₀ units of virus, with a veterinary acceptable carrier and optionally an adjuvant, in a volume of between 0.1 and 5 ml and preferably about 2 ml. The precise amount of a virus in a vaccine composition effective to provide a protective effect can be determined by a skilled veterinary physician. Veterinary acceptable carriers suitable for use in vaccine compositions can be any of those described herein below.

Typically, a vaccine contains between about 1 x 10² and about 1 x 10¹⁰ plaque or colony forming units of virus, with a veterinary acceptable carrier and an adjuvant, in a volume of between 0.1 and 5 ml and preferably about 2 ml. The precise amount of a virus in a vaccine composition effective to provide a protective effect can be determined by a skilled veterinary physician. Veterinary acceptable carriers suitable for use in vaccine compositions can be any of those described hereinbelow. The typical route of administration will be intramuscular or subcutaneous injection of between about 0.1 and about 5 ml of vaccine. The vaccine compositions of the present invention can also include additional active ingredients such as other vaccine compositions against BVDV, e.g., those described in WO 95/12682, WO 99/55366, U.S. Patent No. 6,060,457, U.S. Patent No. 6,015,795, U.S. Patent No. 6,001,613, and U.S. Patent No. 5,593,873.

Vaccination can be accomplished by a single inoculation or through multiple inoculations. If desired, sera can be collected from the inoculated animals and tested for the presence of antibodies to BVD virus. In another embodiment of the present invention, the vaccine compositions are used in treating BVDV testicular infections. Accordingly, the present invention provides methods of controlling or preventing infections in animal subjects caused by types 1 or 2 BVD viruses, or a combination of type 1 and type 2, by administering to an animal, an effective amount of a BVDV virus of the present invention. In another embodiment the vaccine compositions of the present invention are effective for the improvement of herd fertility, and for the reduction of the risk of testicular infection among susceptible male animals.

In practicing the present methods, a vaccine composition of the present invention is administered to cattle preferably via intramuscular or subcutaneous routes, although other routes of administration can be used as well, such as e.g., by oral, intranasal (e.g. aerosol or other needleless administration), intra-lymph node, intradermal, intraperitoneal, rectal or vaginal administration, or by a combination of routes. Intramuscular administration in the neck region of the animal is preferred. Boosting regimens may be required and the dosage regimen can be adjusted to provide optimal immunization.

By "immunogenic" is meant the capacity of a BVD virus to provoke an immune response in an animal against type 1 or type 2 BVD viruses, or against both type 1 and type 2 BVD viruses. The immune response can be a cellular immune response mediated primarily by cytotoxic T-cells, or a humoral immune response mediated primarily by helper T-cells, which in turn activates B-cells leading to antibody production.

According to the present invention, the viruses are preferably attenuated by serial passages in cell culture prior to use in an immunogenic composition. The methods of modification are well known to those skilled in the art.

The vaccine compositions used in the methods of the present invention can also include additional active ingredients such as other immunogenic compositions against BVDV, e.g., those described in copending Application Serial No. 08/107,908, WO 95/12682, WO 99/55366, U.S. Patent No. 6,060,457, U.S. Patent No. 6,015,795, U.S. Patent No. 6,001,613, and U.S. Patent No. 5,593,873.

In addition, the objectives of the present invention can be accomplished by administering other antigens than BVDV types 1 and/or 2, (a "combination vaccine") such antigens include but are not limited to, BRSV, BHV-1, PIV3, *Leptospira canicola, Leptospira grippotyphosa, Leptospira borgpetersenii hardio-prajitno, Leptospira icterohaemmorrhagia, Leptospira interrogans pomona, Leptospira borgpetersenii hardjo-bovis, Leptospira bratislava, Campylobacter fetus Mannheimia (Pasteurella) haemolytica, Pasteurella multocida, Mycobacterium bovis,* and *Mycobacterium dispar.* In several preferred embodiments the source of the combination vaccine is Bovi-Shield^{®} GOLD^{™} IBR-BVD, Bovi-Shield^{®} GOLD^{™} 3, Bovi-Shield^{®} GOLD^{™} 5, Bovi-Shield^{®} GOLD^{™} IBR-BVD-BRSV-LP, Bovi-Shield^{®} GOLD^{™} FP 5 L5, Bovi-Shield^{®} GOLD^{™} FP 5 VL5 or Preguard^{®} GOLD FP 10 (Pfizer, Inc.).

In addition, the immunogenic and vaccine compositions employed in the methods of the present invention can include one or more veterinary-acceptable carriers. As used herein, "a veterinary-acceptable carrier" includes any and all solvents, dispersion media, coatings, adjuvants, stabilizing agents, diluents, preservatives, antibacterial and antifungal agents, isotonic agents, adsorption delaying agents, and the like. Diluents can include water, saline, dextrose, ethanol, glycerol, and the like. Isotonic agents can include sodium chloride, dextrose, mannitol, sorbitol, and lactose, among others. Stabilizers include albumin, among others. Adjuvants include, but are not limited to, the RIBI adjuvant system (Ribi Inc.), alum, aluminum hydroxide gel, Cholesterol, oil-in water emulsions, water-in-oil emulsions such as, e.g., Freund's complete and incomplete adjuvants, Block co-polymer (CytRx, Atlanta GA), SAF-M (Chiron, Emeryville CA), AMPHIGEN® adjuvant, saponin, Quil A, QS-21 (Cambridge Biotech Inc., Cambridge MA), GPI-0100 (Galenica Pharmaceuticals, Inc., Birmingham, AL) or other saponin fractions, monophosphoryl lipid A, Avridine lipid-amine adjuvant, heat-labile enterotoxin from E. coli (recombinant or otherwise), cholera toxin, or muramyl dipeptide, among many others. The vaccine compositions can further include one or more other immunomodulatory agents such as, e.g., interleukins, interferons, or other cytokines. The vaccine compositions employed in the methods of the present invention can also include Gentamicin and Merthiolate. While the amounts and concentrations of adjuvants and additives useful in the context of the present invention can readily be determined by the skilled artisan, the present invention contemplates compositions comprising from about 50 ug to about 2000 ug of adjuvant and preferably about 500 ug/2 ml dose of the vaccine composition. In another preferred embodiment, the present invention contemplates vaccine compositions comprising from about 1 ug/ml to about 60 ug/ml of antibiotic, and more preferably less than about 30 ug/ml of antibiotic.

The vaccine compositions employed in the methods of the present invention can be made in various forms depending upon the route of administration. For example, the vaccine compositions can be made in the form of sterile aqueous solutions or dispersions suitable for injectable use, or made in lyophilized forms using freeze-drying techniques. Lyophilized vaccine compositions are typically maintained at about 4°C, and can be reconstituted in a stabilizing solution, e.g., saline or and HEPES, with or without adjuvant.

The vaccine compositions of the present invention can be administered to animal subjects to induce an immune response against type 1 or type 2 BVD viruses, or against both type 1 and type 2 BVD viruses. Accordingly, another embodiment of the present invention provides methods of stimulating an immune response against type 1 or type 2 BVD viruses, or against a combination of type 1 and type 2 BVD viruses by administering to an animal subject an effective amount of an immunogenic composition of the present invention described above. By "animal subject" is meant to include any animal that is susceptible to BVDV infections, such as bovine, sheep and swine.

In accordance with the methods of the present invention, a preferred immunogenic composition for administration to an animal subject includes the BVDV cpNADL virus and/or the BVDV cp53637 virus. An immunogenic composition containing a BVDV virus, preferably modified live by serial passage in culture, is administered to a cattle preferably via intramuscular or subcutaneous routes, although other routes of administration can be used as well, such as e.g., by oral, intranasal, intra-lymph node, intradermal, intraperitoneal, rectal or vaginal administration, or by a combination of routes.

Immunization protocols can be optimized using procedures well known in the art. A single dose can be administered to animals, or, alternatively, two or more inoculations can take place with intervals of two to ten weeks. Depending on the age of the animal, the immunogenic or vaccine composition can be readministered. For example, the present invention contemplates the vaccination of healthy cattle prior to six months of age and revaccination at six months of age. In another example, the present invention contemplates the vaccination of prebreeding cattle at about 5 weeks prebreeding (or prior to being added to a herd) and optionally again at about 2 weeks prebreeding or during gestation to protect a fetus against infection caused by BVDV Types 1 and 2. Single doses of the compositions of the present invention can also be administered about 3 to 4 weeks after a first dose. Semiannual revaccination with a single dose of the combination vaccine is also contemplated to prevent BVDV fetal infection.

The extent and nature of the immune responses induced in the cattle can be assessed by using a variety of techniques. For example, sera can be collected from the inoculated animals and tested for the presence of antibodies specific for BVDV viruses, e.g., in a conventional virus neutralization assay.

The term "effective amount" refers to an amount of combination vaccine sufficient to elicit an immune response in the animal to which it is administered. The immune response may comprise, without limitation, induction of cellular and/or humoral immunity. The amount of a vaccine that is therapeutically effective may vary depending on the particular virus used, the condition of the cattle and/or the degree of infection, and can be determined by a veterinary physician.

### Inactivated (Partial or Whole Cell) and Modified Live Vaccines

Inactivated or modified live vaccines for use in the method of the present invention can be prepared using a variety of methods which are known in the art.

For example, BVDV isolates can be obtained directly from infected cow uteri using known techniques.

BVDV isolates can be attenuated using a variety of known methods including serial passage, for example. In addition to modified live viral isolates, a vaccine product employed in the methods of the present invention can also include an appropriate amount of one or more commonly used adjuvants. Suitable adjuvants may include, but are not limited to: mineral gels, e.g., aluminum hydroxide; surface active substances such as lysolecithin; glycosides, e.g., saponin derivatives such as Quil A or GPI-0100; pluronic polyols; polyanions; non-ionic block polymers, e.g., Pluronic F-127 (B.A.S.F., USA); peptides; mineral oils, e.g. Montanide ISA-50 (Seppic, Paris, France), carbopol, Amphigen, Amphigen Mark II (Hydronics, USA), Alhydrogel, oil emulsions, e.g. an emulsion of mineral oil such as BayoIF/Arlacel A and water, or an emulsion of vegetable oil, water and an emulsifier such as lecithin; alum; bovine cytokines; cholesterol; and combinations of adjuvants. In a preferred embodiment, the saponin containing oil-in-water emulsion is conventionally microfluidized.

A particularly preferred source of BVDV type 1 and 2, for use in the method of the present invention is the Bovi-Shield^{®} GOLD^{™} line of vaccine products (PFIZER INC.), containing BVDV strain NADL (acquired from the National Animal Disease Center (NADC), USDA, Ames, IA) and BVDV type 2 strain cp BVDV strain 53637 (Univ. Guelph, Guelph, Ont.) (ATCC No. PTA-4859).

Preferably, the strains NADL and 53637 are modified live strains. In accordance with the present invention, the strains of the present invention can be adjuvanted with a commercially available adjuvant, preferably, Quil A-Cholesterol-Amphigen (Hydronics, USA). A preferred dose of the immunogenic and vaccine compositions of the present invention is about 2.0 ml. Preservatives can be included in the compositions employed in the methods of the present invention. Preservatives contemplated by the present invention include gentamicin and merthiolate. A carrier can also be added, preferably, PBS. Preparation of modified live vaccines, such as by attenuation of virulent strains by passage in culture, is known in the art.

Modified live BVDV isolates can also be combined with the following bacteria and viruses, including but not limited to, bovine herpesvirus type 1 (BHV-1), bovine respiratory syncitial virus (BRSV), parainfluenza virus (PIV3), *Leptospira canicola, Leptospira grippotyphosa, Leptospira borgpetersenii hardio-prajitno, Leptospira icterohaemmorrhagia, Leptospira interrogans pomona, Leptospira borgpetersenii hardjo-bovis, Leptospira Bratislava, Campylobacterfetus, Mannheimia (Pasteurella) haemolytica, Pasteurella multocida, Mycobacterium bovis,* and *Mycobacterium dispar.*

### Dosing and Modes of Administration

According to the present invention, an effective amount of a BVDV or combination vaccine administered to susceptible male animals provides effective immunity against testicular infection associated with type and 2 BVDV. In one embodiment, the vaccine is administered to calves in two doses at an interval of about 3 to 4 weeks. For example, the first administration is performed when the animal is about 1 to about 3 months of age. The second administration is performed about 1 to about 4 weeks after the first administration of the combination vaccine.

In another preferred embodiment, an administration, is performed about 4 to 5 weeks prior to animal breeding or prior to admittance to an artificial insemination facility. Administration of subsequent vaccine doses is preferably done on an annual basis. In another preferred embodiment, animals vaccinated before the age of about 6 months should be revaccinated after 6 months of age. Administration of subsequent vaccine doses is preferably done on an annual basis, although bi-annual and semiannual subsequent vaccine doses are also contemplated by the present invention.

The amount of vaccine that is effective depends on the ingredients of the vaccine and the schedule of administration. Typically, when a modified live BVDV preparation is used in a vaccine, an amount of the vaccine containing about 10² to about 10'° TCID₅₀ units per dose of BVDV, and preferably about 10⁴ to about 10⁷ TCID₅₀ units per dose of types 1 and 2 BVDV is effective when administered once to susceptible male animals. Preferably, a vaccine that provides effective immunity contains about 10⁴ to 10⁷ TCID₅₀ units/dose of types 1 and 2 BVDV and more preferably, about 10⁵ TCID₅₀ units/dose, when administered once to susceptible animals. Administration of subsequent vaccine doses is preferably done on an annual basis. Animals vaccinated before the age of about 6 months should be revaccinated after 6 months of age. Administration of subsequent vaccine doses is preferably done on an annual basis.

According to the present invention, when the preferred product, Bovi-Shield^{®} GOLD^{™} 5 (Pfizer, Inc.), is administered, the product is administered preferably once, in the amount of about 0.1 ml to about 5.0 ml, preferably about 1.5 ml to about 2.5 ml, and more preferably, about 2 ml. Administration of subsequent vaccine doses is preferably done on an annual basis. Animals vaccinated before the age of about 6 months should be revaccinated after 6 months of age. Administration of subsequent vaccine doses is preferably done on an annual basis.

In accordance with the present invention, administration can be achieved by known routes, including the oral, intranasal, topical, transdermal, and parenteral (e.g., intravenous, intraperitoneal, intradermal, subcutaneous or intramuscular). A preferred route of administration is intramuscular or subcutaneous administration.

The present invention also contemplates a single primary dose followed by annual revaccination, which eliminates the necessity of administration of additional doses to calves prior to annual revaccination in order to generate and/or maintain immunity against infection.

The vaccines administered in accordance with the present invention can include additional components, such as an adjuvant (e.g., mineral gels, e.g., aluminum hydroxide; surface active substances such as Cholesterol, lysolecithin; glycosides, e.g., saponin derivatives such as Quil A, QS-21 or GPI-0100; pluronic polyols; polyanions; non-ionic block polymers, e.g., Pluronic F-127; peptides; mineral oils, e.g. Montanide ISA-50, carbopol, Amphigen®, Alhydrogel, oil emulsions, e.g. an emulsion of mineral oil such as BayolF/Arlacel A and water, or an emulsion of vegetable oil, water and an emulsifier such as lecithin; alum; bovine cytokines; and combinations of adjuvants.).

According to the present invention, the administration of an effective amount of a vaccine administered to susceptible male animals at approximately 3 months of age provides effective immunity against testicular infection.

In a preferred embodiment, the vaccine is administered intramuscularly. In another preferred embodiment, the vaccine is administered subcutaneously. Moreover, it is preferred that the vaccine dose comprise about 1 ml to about 7ml, and preferably about 2 ml, each ml containing about 10² to about 10¹⁰ TCID₅₀ units/per dose of virus. The combination vaccine is desirably administered twice to the animal; once at about 1 to about 3 months of age, and once at about 5 to 3 weeks later. The present invention also contemplates annual revaccinations with a single dose.

### Identification of Animals at Increased Risk of BVDV Infection

The invention further comprises a method of preventing testicular BVDV infection in a male animal susceptible to BVDV infection comprising:
a) identifying an animal with an increased risk of BVDV testicular infection; and
b) administering to the animal an effective amount of a vaccine selected from the group consisting of a killed type 1 BVDV vaccine , a killed type 2 BVDV vaccine, a modified live type 1 BVDV vaccine, and a modified live type 2 BVDV vaccine.

To identify an animal suffering from an increased risk of BVDV infection the skilled practitioner recognizes that an animal suffers from an elevated risk of infection when BVDV infection is introduced into a previously uninfected herd or wherein an a susceptible animal is in contact with a another susceptible animal with a BVDV infection. BVDV is spread from animal to animal in a fecal-oral manner. The viral load needed to provoke symptomatic infection is correlated with the type and strain of BVD virus and this is correlated with the rapidity of spread throughout the herd. Infected animals can be identified by symptomology. Common manifestations of BVDV infection can include: abortion storms, infertility, irregular heat cycles, early embryonic deaths, fetal mummification, immune-suppression, dysentery, thrombocytopenia, and cerebral hypoplasia. Symptoms of the disease are usually preceded by leukopenia, and testing efforts to date have focused on identifying this effect.

Serological studies have shown that a high percentage of cattle infected with BVDV, including those considered to be persistently infected (PI), remain clinically asymptomatic. Therefore a preferred method of identifying infected animals is to detect the presence of the virus itself rather than relying on symptomology. Several different test methods have been developed for the detection of BVDV, and/or the detection of BVDV-infected animals. These test methods include: reverse transcription-polymerase chain reaction, enzyme-linked immunoassay (ELISA), standard virus isolation techniques, and immunohistochemistry (Haines et al., "Monoclonal Antibody-Based Immunohistochemical Detection of Bovine Viral Diarrhea Virus in Formalin- Fixed, Paraffin-Embedded Tissues," Vet. Pathol., 29:27-32 (1992)).

Both PCR and virus isolation techniques, owing to their inherent sensitivity, are each capable of detecting very low levels of BVDV. Immunohistochemistry on tissue samples, such as ear notch biopsy samples, is an effective technique for detecting PI animals as well ELISA technology, although somewhat less sensitive, is well suited as a broad-based diagnostic tool for detecting BVDV infection in animals, because it is inexpensive, yields results in a short period of time, and does not require highly trained technicians and a highly specialized laboratory facility.

ELISA methods for detection of BVDV infection are described in the literature See U.S. Pat. No. 6,174,667 and WO 99/15900 to Huchzermeier et al. and US Patent Application Publication 20030143573 and have been compared to other methods, "Comparison of an Antigen Capture Enzyme-Linked Assay with Reverse Transcription- Polymerase Chain Reaction and Cell Culture Immunoperoxidase Tests for the Diagnosis of Ruminant Pestivirus Infections," Vet. Microbiol., 43:75-84 (1995)).

The present invention is further illustrated, but not limited by the following examples.

### Example 1

### Testicular Protection: Study Overview

The Bovi-Shield^{®} GOLD^{™} 5 vaccine line, formulated with both type 1 BVDV and type 2 BVDV, was introduced to the cattle industry in November 2003 to optimize the level of fetal protection that vaccination provides against type 2 BVDV. Reported here are the results of a prelicensing efficacy study assessing whether prepuberal vaccination with Bovi-Shield^{®} GOLD^{™} 5 was effective in preventing testicular infection in the face of a severe type 2 BVDV challenge.¹⁰ versus a placebo (Bovi-Shield^{®} IBR-PI3-BRSV).

All bull calves (n = 17) enrolled in a more extensive type 2 BVDV challenge study were further evaluated to determine whether vaccination with Bovi-Shield GOLD^{™} 5^{®} effectively prevented testicular infection with BVDV. All calves in the initial study were 3- to 4-month-old colostrum-deprived beef calves (male and female). At 28 days after vaccination with a formulation containing minimum immunizing doses, (Minimum immunizing dose levels are established prior to licensing of a vaccine and reflect a lower volume of antigenic virus than is present in the finished product. Determination of the minimum immunizing dose helps ensure that when a product is used at release levels it will consistently stimulate adequate protection against disease.) of both type 1 BVDV and type 2 BVDV, all vaccinates and placebo control calves were intranasally challenged with noncytopathic type 2 BVDV strain 24515. Strain 24515 was isolated in Canada from a severe BVD outbreak that killed more than 40% of cattle in affected herds. Following challenge, all 10 control calves developed severe disease characterized by prolonged viremia (9 to 14 days), fever ranging from 105.6° F to 107.2° F for 4 to 9 days, leukopenia (1 to 9 days), thrombocytopenia (< 100,000 per µL), morbidity (ranging from 4 to 9 days), and high mortality (7 of 10 (70%) controls died). In contrast, only 1 vaccinate developed viremia, 6 were febrile for 1 or 2 days, 1 was leukopenic for 1 day, none were thrombocytopenic, and none died. Altogether, 18 of 20 vaccinates remained healthy throughout the study with only 2 calves showing depression on 1 day. These two observations were not associated with previous or concurrent viremia, fever, leukopenia, or thrombocytopenia.¹¹

Evaluations for BVDV testicular infection were initiated approximately 2 weeks after challenge. As Table 1 shows, testicle samples were collected at necropsy from 2 placebo controls on Study Day 41 and biopsy samples from each of the remaining 5 control and 10 Bovi-Shield^{®} GOLD^{™} 5 vaccinates on Day 42. A second sample was also obtained from 7 of the 10 Bovi-Shield^{®} GOLD^{™} 5 vaccinates that were still available on Day 56. All samples were tested for BVDV using tissue culture isolation, nucleic acid amplification (RT-nPCR), and immunohistochemical testing methods. Personnel collecting and testing the testicular samples had no knowledge of treatment group assignments.

| Table 1-Study design: Testicular protection following BVDV Type 2 challenge | | | | | | |
|---|---|---|---|---|---|---|
| **Treatment** | **No. Bull** | **Intramuscular Vaccination** | | **Intranasal Challenge*** | | |
| **Group** | **Calves** | **Day** | **Dose** | **Day** | **Dose** | **Sampling Day** |
| Placebo controls | 7 | 0 | 2-mL | 28 | 5-mL | 41, 42^{†} |
| Bovi-Shield^{®} GOLD^{™} 5 | 10 | 0 | 2-mL | 28 | 5-mL | 42,56^{‡} |

| | | | | | | |
|---|---|---|---|---|---|---|
| *Isolate 24515 was obtained from the University of Guelph, Guelph, Ontario, Canada. ^{†}two of 7 placebo vaccinated bull calves were sampled at necropsy on Day 41 and the remaining 5 calves were sampled on Day 42. ^{‡}µAll 10 bull calves were sampled on Day 42 and a second sample was obtained from 7 calves on Day 56. | | | | | | |

Virus isolation and PCR assays were performed at the Auburn University Veterinary Pathobiology and Clinical Sciences Laboratory, and immunohistochemical analysis at the University of Nebraska-Lincoln Veterinary Diagnostic Center. Data were analyzed by a representative of Pfizer Animal Health, Veterinary Medicine and Research, Biometrics, Technology and Quality, with a categorical procedure (SAS/STAT Software Changes and Enhancements through Release 6.12, SAS Institute, Cary, NC, or SAS/STAT User's Guide Version 8 and SAS Procedures Guide Version 8). Fisher's Exact Test was used to compare the proportion of animals in each treatment group with at least one BVDV positive test result. Descriptive statistics were calculated as appropriate.

### Results

Table 2 and Figure 1 summarize the treatment group percentages for BVDV detection in the testicular biopsy specimens. BVDV nucleic acid or antigen was detected in 6 of 7 (85.7%) testicular specimens collected from the placebo-vaccinated and challenged bulls. In contrast, no BVDV nucleic acid or antigen was detected in any (0.0%) of the testicular specimens collected from challenged bull calves vaccinated with Bovi-Shield^{®} GOLD^{™}, a significant (P ≤ 0.05) difference.

| Table 2-Summary BVDV assay results for testicular specimens | | | | | | |
|---|---|---|---|---|---|---|
| | **No.** | **No. BVDV Positive** | | | **BVDV** | **% Calves** |
| **Group** | **Calves** | **VI** | **PCR** | **IHC** | **Positive** | **Positive** |
| Placebo | 7 | 5 | 6 | 4 | 6 | 85.7%^{a} |
| *Bovi-Shield GOLD* | 10 | 0 | 0 | 0 | 0 | 0.0%^{b} |

| | | | | | | |
|---|---|---|---|---|---|---|
| VI = virus isolation. PCR = polymerase chain reaction, IHC = immunohistochemistry ^{a,b}Percents within a column with different lower-case superscripts are significantly (P ≤ 0.05) different. | | | | | | |

### Conclusion and Discussion

Study results demonstrated both the safety and efficacy of vaccinating bulls with Bovi-Shield^{®} GOLD^{™} 5. Vaccine formulated with minimum immunizing dose levels of type 1 and 2 BVDV not only did not cause testicular infection but also effectively protected prepuberal bull calves against testicular infection following severe challenge with type 2 BVDV.

Use of Bovi-Shield^{®} GOLD^{™} 5 in prepuberal bulls may be an important component of BVD control programs in cow-calf and dairy operations. Timely vaccination can help protect bull calves against acute infections that have been associated with transient and persistent testicular infection and subsequent transmission of BVDV in semen to susceptible cows. Additionally, vaccination of prepuberal bulls to prevent acute postpuberal BVDV infection may help maintain semen quality, which has been shown to be affected (decreased motility and morphologic abnormalities) during the first 60 days after acute BVDV infection.⁹

Although the results here demonstrate successful protection after challenge with type 2 BVDV it would be expected that similar results would be observed after challenge with type 1 BVDV.

### Example 2

### Testicular Protection against type 1 BVDV challenge-Study Overview

A study was undertaken to assess the efficacy of Bovi-Shield^{®} GOLD^{™} 5 in preventing testicular infection in the face of a severe type 1 BVDV challenge. The design of this study was a generalized randomized block design with one-way treatment structure. All study laboratory personnel were masked from treatment information.

Forty-five (45) peri-pubertal intact beef-breed bull calves were allotted in to the study. The calves were from 9 to 15 months of age. All animals were seronegative to BVD virus Types 1 and 2, negative for BVD virus isolation in serum and negative by reverse transcriptase-nested polymerase chain reaction (RT-nPCR) from serum. On Day 42, the least squares means of body weights for calves in the placebo group (n=23) and the test group (n=22) were 625.7 ± 30.94 lbs and 613.5 ± 35.96 lbs, respectively. Animals were vaccinated with either placebo (Bovi-Shield^{®} IBR-PI3-BRSV) or Bovi-Shield^{®} GOLD^{™} 5 (IBR-BVDV 1-BVDV2-P13-BRSV).

At 28 days after vaccination with a formulation containing minimum immunizing doses all vaccinates and placebo control calves were intranasally challenged with noncytopathic type la BVDV virus strain SD-1 isolated at Auburn University. Intranasal challenge was performed by hyperventilating the bulls for 30 seconds via placing a plastic bag over the nostrils and then instilling 5 mL of virus grown in MEM with Earle's salts supplemented with 10% (vol/vol) equine serum sodium bicarbonate (0.75 mg/mL), L-glutamine (0.29 mg/mL) and antibiotics (100 units penicillin G, 100 µg streptomycin and 0.25 µg amphotericin B/mL). Equine serum was free of BVD virus as determined by virus isolation and RT-nPCR.

Evaluations for BVDV testicular infection were performed at day 42 and 93. The basic study design is outlined in Table 3 below.

| Table 3-Study design: Testicular protection following BVDV Type 1 challenge | | | | | | |
|---|---|---|---|---|---|---|
| **Treatment** | | **Intramuscular Vaccination** | | **Intranasal Challenge*** | | |
| **Group** | **No. Bull Calves** | **Day** | **Dose** | **Day** | **Dose** | **Sampling Day** |
| Placebo controls | 23 | 0 | 2-mL | 28 | 5-mL | 42, 93 |
| Bovi-Shield^{®} GOLD^{™} 5 | 22 | 0 | 2-mL | 28 | 5-mL | 42, 93 |

Following challenge, serum animal in both groups was tested for the presence of virus. As tested by serum virus isolation, 18 calves in the placebo group were viremic on Day 34 Nine of these 18 were also viremic on Day 35. Five calves in the Bovi-Shield^{®} GOLD^{™} 5 group were viremic on Day 34 only. No other virus isolation tests were positive for calves from either treatment group on any study day.

RT-nPCR tests was also used to determine if calves were viremic. For the placebo group, there were 3, 9, 12 and 6 calves with positive results on Days 34, 35, 36 and 37 respectively. For the Bovi-Shield^{®} GOLD^{™} 5 group, there were 1, 1,2, and 1 calves with positive results on Days 34, 35, 36 and 37, respectively. No other serum RT-nPCR tests were positive for calves from either treatment group on any study day.
Least square mean rectal temperatures were measured for both groups. Only on Day 36 were the means (104.0 °F of for placebo and 102.9 °F for the Bovi-Shield GOLD 5 group) significantly different
(*P ≤* 0.05).

### Testicular Protection Results

Results of testing conducted on Day 93 testicular biopsy samples are shown in Table 4. Samples from 6 of 23 calves (26.1 %) in the placebo group were positive on RT-nPCR testing, while all of the samples from the 22 calves in the Bovi-Shield^{®} GOLD^{™} 5 group were negative. All biopsy samples from both the placebo and Bovi-Shiele GOLD^{™} 5 groups were negative for virus isolation. Biopsy samples from 5 of 23 calves (21.7%) in the placebo group and 0 of 22 calves in the Bovi-Shield^{®} GOLD^{™} 5 group were positive on IHC testing.

All semen samples calves in both treatment groups were negative for RT-nPCR testing for BVD virus on Day 42 (Table 4). On Day 93, 10 of 23 (43.5%) calves in the placebo group were positive on the RT-nPCR test but all 22 calves in the Bovi-Shield^{®} GOLD^{™} 5 group were negative. No virus was isolated from any semen sample from either treatment group on Day 42 or Day 93 (Table 4).

In the placebo group 10 of 23 calves were positive for at least one test. Four of these ten calves were positive for only one test (semen RT-PCR on Day 93). One calf was positive for two tests (testicular biopsy RT-nPCR and semen RT-nPCR on Day 93). Five calves were positive for three tests (testicular biopsy RT-nPCR, semen RT-nPCR and biopsy IHC on Day 93).

| Table 4-Summary of BVDV assay results for testicular specimens and semen samples | | | | | | | | | | |
|---|---|---|---|---|---|---|---|---|---|---|
| **Group** | **No. Calves** | **No. BVDV Positive†** | | | **No. BVDV Positive ‡** | | **No. BVDV Positive*** | | **BVDV Positive** | **% Calves Positive** |
| | | **VI** | **PCR** | **IHC** | **VI** | **PCR** | **VI** | **PCR** | | |
| Placebo | 23 | 0 | 6 | 5 | 0 | 0 | 0 | 10 | 10 | 43.5%^{a} |
| Bovi-Shield GOLD | 22 | 0 | 0 | 0 | 0 | 0 | 0 | 0 | 0 | 0%^{b} |

| | | | | | | | | | | |
|---|---|---|---|---|---|---|---|---|---|---|
| VI = virus isolation, PCR = polymerase chain reaction, IHC = immunohistochemistry ^{a,b}Percents within a column with different lower-case superscripts are significantly (P ≤ 0.0002) different. ^{†} Day 93 Testicular Biopsy Samples ^{‡} Day 42 Semen Samples * Day 93 Semen Samples | | | | | | | | | | |

For each animal, the presence/absence of BVD virus in either semen or testicular biopsies at any time-point was determined, summarized by treatment group, and analyzed using Fisher's Exact test as described in Example 1.

### Conclusion

In this study, vaccination of bull calves with Bovi-Shield^{®} GOLD^{™} 5 prevented persistent testicular infection with BVD virus Type 1 and subsequent shedding of virus in semen. The contrast between placebo and vaccinated animals for at least one positive test by the two-tailed Fisher's Exact Test was significant (P = 0.0002).

The present invention is not limited in scope by the specific embodiments described, which are intended as single illustrations of individual aspects of the invention. Functionally equivalent compositions and methods are within the scope of the invention. Indeed, various modifications of the invention, in addition to those shown and described herein, will become apparent to those skilled in the art from the foregoing description. Such modifications are intended to fall within the scope of the appended.

## Claims

1. Use of an effective amount of an antigen selected from the group consisting of an inactivated type 1 bovine viral diarrhea virus (BVDV), an inactivated type 2 BVDV, a modified live type 1 BVDV, and a modified live type 2 BVDV, for the manufacture of a vaccine for preventing testicular BVDV infection in a susceptible male animal.

2. The use of claim 1, wherein the animal is selected from the group consisting of a bull, a ram, and a boar.

3. The use of claim 2, wherein the animal is a bull.

4. The use of claim 1, wherein the vaccine comprises both a modified live type 1 BVDV and a modified live type 2 BVDV.

5. The use of claim 4, wherein at least one of the modified live BVDV is derived from a cytopathogenic virus.

6. The use of claim 4 wherein at least one of the modified live BVDV is derived from a non-cytopathogenic virus.

7. The use of claim 4, wherein both of the modified live BVDV are derived from a cytopathogenic virus.

8. The use of any of claims 1-7, wherein the vaccine comprises one or more additional antigens selected from the group consisting of Bovine Herpes Virus (BHV-1), Parainfluenza Virus Type 3 (PIV3), Bovine Respiratory Syncytial Virus (BRSV), *Leptospira canicola, Leptospira grippotyphosa, Leptospira borgpetersenii hardjo-prajitno, Leptospira icterohaemmorrhagia, Leptospira interrogans pomona, Leptospira borgpetersenii hardjo-bovis, Leptospira bratislava, Campylobacter fetus, Mannheimia (Pasteurella) haemolytica, Pasteurella multocida, Mycobacterium bovis,* and *Mycobacterium dispar.*

9. The use of claim 8, wherein said additional antigens comprise Bovine Herpes Virus (BHV-1), Parainfluenza Virus Type 3 (PIV3), and Bovine Respiratory Syncytial Virus (BRSV).

## Patentansprüche

1. Verwendung einer wirksamen Menge von mindestens einem Antigen, ausgewählt aus der Gruppe, bestehend aus einem inaktivierten Typ 1-BVDV, einem inaktivierten Typ 2-BVDV, einem modifizierten Lebendtyp 1-BVDV und einem modifizierten Lebendtyp 2-BVDV und Kombinationen davon, zur Herstellung eines Impfstoffs zum Verhindern oder kontrollieren von testikulärer BVDV-Infektion bei einem anfälligen männlichen Lebewesen.

2. Verwendung nach Anspruch 1, wobei das Lebewesen aus der Gruppe, bestehend aus einem Bullen, einem Schafbock und einem Eber, ausgewählt ist.

3. Verwendung nach Anspruch 2, wobei das Lebewesen ein Bulle ist.

4. Verwendung nach Anspruch 1, wobei der Impfstoff sowohl einen modifizierten Lebendtyp 1-BVDV als auch einen modifizierten Lebendtyp 2-BVDV umfasst.

5. Verwendung nach Anspruch 4, wobei mindestens einer von den modifizierten Lebend-BVDV von einem cytopathogenen Virus abgeleitet ist.

6. Verwendung nach Anspruch 4, wobei mindestens einer von den modifizierten Lebend-BVDV von einem nicht-cytopathogenen Virus abgeleitet ist.

7. Verwendung nach Anspruch 4, wobei beide von den modifizierten Lebend-BVDV von einem cytopathogenen Virus abgeleitet sind.

8. Verwendung nach einem der Ansprüche 1 bis 7, wobei der Impfstoff ein oder mehrere zusätzliche Antigene, ausgewählt aus der Gruppe, bestehend aus Rinder-Herpes-Virus (BHV-1), Parainfluenza-Virus Typ 3 (PIV-3), Rinder-Respiratory-Syncytial-Virus (BRSV), Leptospira canicola, Leptospira grippotyphosa, Leptospira borgpetersenii hardjo-prajitno, Leptospira icterohaemmorrhagia, Leptospira interrogans pomona, Leptospira borgpetersenii hardjo-bovis, Leptospira Bratislava, Campylobacter fetus, Mannheimia (Pasteurella) haemolytica, Pasteurella multocida, Mycobacterium bovis und Mycobacterium dispar, umfasst.

9. Verwendung nach Anspruch 8, wobei die zusätzlichen Antigene Rinder-Herpes-Virus (BHV-1), Parainfluenza-Virus Typ 3 (PIV3) und Rinder-Respiratory-Syncytial-Virus (BRSV) umfassen.

## Revendications

1. Utilisation d'une quantité efficace d'au moins un antigène choisi dans le groupe consistant en un BVDV de type 1 inactivé, un BVDV de type 2 inactivé, un BVDV de type 1 vivant modifié et un BVDV de type 2 vivant modifié ainsi que leurs associations, pour la production d'un vaccin destiné à la prévention ou à la lutte contre l'infection testiculaire par le BVDV chez un animal mâle sensible.

2. Utilisation suivant la revendication 1, dans laquelle l'animal est choisi dans le groupe consistant en un taureau, un bélier et un verrat.

3. Utilisation suivant la revendication 2, dans laquelle l'animal est un taureau.

4. Utilisation suivant la revendication 1, dans laquelle le vaccin comprend à la fois un BVDV de type 1 vivant modifié et un BVDV de type 2 vivant modifié.

5. Utilisation suivant la revendication 4, dans laquelle au moins un des BVDV vivants modifiés est dérivé d'un virus cytopathogène.

6. Utilisation suivant la revendication 4, dans laquelle au moins un des BVDV vivants modifiés est dérivé d'un virus non cytopathogène.

7. Utilisation suivant la revendication 4, dans laquelle les deux BVDV vivants modifiés sont dérivés d'un virus cytopathogène.

8. Utilisation suivant l'une quelconque des revendications 1 à 7, dans laquelle le vaccin comprend un ou plusieurs antigènes supplémentaires choisis dans le groupe consistant en le virus d'herpès bovin (BHV-1), le virus parainfluenza de type 3 (PIV3), le virus syncytial respiratoire bovin (BRSV), *Leptospira canicola, Leptospira grippotyphosa, Leptospira borgpetersenii hardjo-prajitno, Leptospira icterohaemmorrhagiae, Leptospira interrogans pomona, Leptospira borgpetersenii hardjo-bovis, Leptospira bratislava, Campylobacter fetus, Mannheimia (Pasteurella) haemolytica, Pasteurella multocida, Mycobacterium bovis* et *Mycobacterium dispar.*

9. Utilisation suivant la revendication 8, dans laquelle lesdits antigènes supplémentaires comprennent le virus d'herpès bovin (BHV-1), le virus parainfluenza de type 3 (PIV3) et le virus syncytial respiratoire bovin (BRSV).
